# EUROPEAN PATENT APPLICATION

(11) **EP 4 450 113 A1**
(43) Date of publication of application: **23.10.2024**
(21) Application number: 24169859.6
(22) Date of filing: 12.04.2024
(51) Int. Cl.: A61M 29/00, A61B 17/42, A61B 1/32

(54) **VAGINAL SPECULUM**

(30) Priority: 17.04.2023 IT 202300007362
(71) Applicant: Universita' IUAV di Venezia, 30135 Venezia (IT)
(72) Inventor: SCALA, Emma, 30135 VENEZIA (IT); ROMERO, Maximiliano Ernesto, 30135 VENEZIA (IT)
(74) Representative: De Filippis, Sara

(57) **Abstract**

The invention concerns an instrument (100) for vaginal dilation, comprising two valves (1) joined by a handle (2), characterized in that it presents a clamshell structure, and in that the handle (2) presents a central through opening (6) between two side elements (7) joined at a first junction end (4) and at a second junction end (5), the valves (1) being directly connected transversally to the side elements (7).

## Description

The present invention falls within the field of medical devices.

### Field of the invention

In particular, it regards an instrument used during the gynecological examination to inspect the vaginal cavity.

In the following the description will be aimed at a vaginal speculum, but it is clear that it should not be considered limited to this specific use.

### Prior Art

The shape of the retractors has remained unchanged since 1870, when Edward Cusco reinterpreted the instrument by designing the Cusco's Speculum, which is still used today.

The Cusco's speculum remains an intimidating, uncomfortable and painful device for many patients. Its aim is to exert a retracting force on the upper and lower walls of the vagina, which are more sensitive due to the presence of various organs: the female genital system, in the upper area there are the bladder and the clitoral nerves, and in the lower area there is the colon.

Recently, the interest in addressing this issue has increased and some redesign projects have been developed which have introduced changes to the material or mechanism, while maintaining the typical shape of the instrument.

Many of these innovative projects remained as concepts and did not reach commercialization. The aim of the present invention is to propose an alternative solution through the design of a speculum that meets the anatomical and perceptive needs of patients and considers the needs of gynecologists.

The equipment used during the examination can be uncomfortable and make women feel uncomfortable, influencing their physical and emotional state.

Furthermore, there are some pathologies that characterize the life of a woman and her relationship with her private parts and that make the gynecological examination difficult to perform.

In general, menopausal women experience significant physical and hormonal problems, but in particular the pathologies are: Dyspareunia, a pathology characterized by genital pain linked to penetration during or after sexual intercourses; endometriosis, a chronic benign inflammation of the female genital organs and of the pelvic peritoneum, wherein the endometrial tissue is positioned in locations other than the physiological one; vulvo-vaginal atrophy, which consists in the progressive modification of the structure of the vaginal and vulvar tissue as a consequence of the lack of estrogens, which lead to a thinning of the vagina walls which become more fragile and less lubricated; and vaginismus, a pathology characterized by hyper-contracture of the vaginal muscles, i.e. involuntary contractions during penetration. In all these cases, the gynecological examination is greatly complicated given the discomfort created in the patient by the Cusco's Speculum.

A speculum is known, called the Hegenberger's Speculum from the name of its creator, according to documents no. IN202117055842 and RU2780010; it is a particular speculum made of plastic material to be inserted into the vagina, without lubricant, which makes it possible a correct and wide opening of the vulva: in this way full visibility and the possibility of having both hands free to operate are guaranteed to the doctor.

However, the Hegemberger's Speculum is used on post-childbirth patients to facilitate the application of sutures. The physical condition of the woman implies that her vaginal pressure is limited, therefore the plastic material used has mechanical characteristics which are effective for these cases.

In detail, it is a speculum adapted to be inserted with the bend of junction between the valves inside the vagina, obstructing the view inside the vaginal canal itself.

Furthermore, all the force for retracting the vagina is concentrated on that single bend of junction, making the impact on the patient more significant.

Other known specula present a plurality of components and straight and rigid lines, which therefore present shapes that are not compatible with the vagina anatomy, in addition to requiring assembly and disassembly in their respective production and disposal steps, with a consequent increase in management costs.

A further category is that of the inflatable specula, such as those of the type described in document no. CA2242738.

All the studies behind these projects start from a common point, i.e. the need to reduce the discomfort and the feeling of vulnerability and the need to improve the intimidating appearance of the speculum.

It is suggested a progressive opening mechanism with a controlled change in diameter: in this way the speculum configuration when entering has small dimensions that change once inserted.

These solutions make it possible to abandon the use of predefined sizes and to guarantee an adaptability depending on the woman. However, many articles in this regard report a common difficulty, i.e. the need to counteract the strong muscular pressure of the vaginal wall and the need to prevent the instrument from collapsing on itself.

In the case of the disposable speculum made of plastic material, its disposal is provided: first a sanitization step is necessary given the infectious risks associated with the device. Some types of plastic material cannot withstand high temperature treatments, so sterilization is carried out using ethylene oxide (EtO): the procedure is dangerous as the gas is flammable and explosive, which is why it follows the ATEX directives (ATEX 2014/34/UE and ATEX 1999/92/EC).

Furthermore, the sterilization issue persists even when using sterilizable plastic materials. In fact, the vaginal speculum cannot be touched by the doctor after use, as it is a hazardous waste. The device used today is made up of three elements in two different materials: this means that it cannot be disassembled after use and the two different materials of which it is made cannot be disposed of correctly.

Fundamentally, the following issues are encountered for the specula currently used.

Issues related to the sizes:
- the sizes of the instrument that are available are not always sufficiently adaptable to the patient's vagina;
- within hospitals, clinics and surgeries of the National Health Service, only two sizes are often available (S and M);
- by using the speculum in size S, which is one of the most used for the pap test, it may be more difficult to identify the cervix;

Issues related to the material:
- several doctors note the waste of plastic material due to the packaging and to the instrument which, after use, are disposed of in the same bin and cannot be recycled as it is waste with a high infectious risk;

Issues related to the female anatomy:
- if the walls are not relaxed, the insertion of the instrument may be perceived as uncomfortable: this issue is not exclusive to anxious or worried women. In a resting state, without sexual arousal, the vaginal canal collapses on itself and is neither dilated nor perfused. The current solution is to instruct women to breathe deeply;
- the use of lubricant is necessary due to the collapsing nature of the vagina in its resting state. However, lubricating gel can only be used if a pap test is not being performed, otherwise, it will alter the results;
- uterus in abnormal positions: accessing the cervix may be challenging and painful for the patient.

Issues related to the valve mechanism:
- some trainees say that, due to the force used, it can be difficult to open it delicately;
- when removing the speculum, an "opening and closing" and "back and forth" movement is performed: this movement is slow and imperceptible for women but is necessary to prevent the walls from being pinched;
- several trainees report difficulties/inconvenience in separating the valves when only one is needed: in fact, doctors perform this operation with gloves and swiftly;
- some doctors often choose not to lock the instrument during examination to prevent causing pain;
- the speculum must be inserted at an oblique angle to prevent the urethra, a very delicate part, from being damaged by the ends of the valves.

Speculum usage issues encountered by the user:
- insertion may be uncomfortable at the level of the vulva;
- the opening can be painful at the level of the vulva: this is because women have more sensitivity in the external part and in the first half of the vaginal tract;
- some women have experienced pinching of the vaginal walls: i.e., when the skin remains stuck between the valves while closing and removing the speculum;
- many women describe this instrument as intimidating and outdated;
- the metal speculum is particularly cold for women; several doctors warm it with water;
- due to the material, the instrument is perceived as hard and resistant.

### Aim of the invention

In the gynecological field, particularly during examination, there are two users to consider: the doctor and the patient. The two roles interact differently with the device: the doctor uses it and the patient experiences it. For this reason, the instrument must meet numerous performance criteria.

Although the patient does not directly use the medical device, the user-centered design takes into account some aspects: qualitative perception, aesthetic appearance, safety and reliability are crucial for reassuring the patient during an examination.

On the other hand, the device in question is a working instrument for the doctor: it must ensure various qualitative and ergonomic performances, as well as ease of use. The doctor must be certain to protect the patient and to use a non-harmful instrument. A medical product that considers the needs of the user-doctor should be a simple and intuitive device.

The main objective of the present invention is to provide a speculum that overcomes the aforementioned drawbacks and, in particular, allows a gynecological examination to be performed and/or experienced in an easy and comfortable manner.

In detail, the objective is to improve the experience of patients, especially young women, during gynecological examinations.

Another objective is to make examinations more bearable, thereby encouraging regular examinations to promote compliance with a prevention plan.

Furthermore, the present invention proposes a redesign of the instrument used in inspection examinations to observe the vagina and cervix, which reduces pain and discomfort for women and is easy for doctors to use.

Finally, the present invention proposes a device designed to optimize both the production and recycling steps.

The gynecological examination can be experienced by women as an event difficult to bear due to various aspects, including the speculum, which affects their emotional state and consequently their physical state.

The objective is to provide the doctor with an alternative instrument to resolve one of the major obstacles of the examination.

In detail, the present invention has the following objectives:
- Ergonomic and comfortable grip for the doctor;
- Shape that facilitates insertion of the instrument;
- Progressive opening of the instrument walls;
- Material compliant with hospital standards;
- Use of comfortable materials for women;
- Allowing the doctor to have full visibility;
- Maintaining the opening of the vaginal canal for carrying out tests (e.g. Pap tests) and observing;
- Resistance to vaginal pressure;
- Device that changes its diameter;
- Moderate opening time;
- Ease of use for doctors;
- Comfort for patients.

### Object of the invention

The object of the invention is therefore an instrument for vaginal dilation, comprising two valves joined by a handle, characterized in that it presents a clamshell structure, and in that the handle presents a central through opening between two side elements joined at a first junction end and at a second junction end, while the valves are directly connected transversally to the side elements.

This allows wide visibility inside the vaginal canal through the opening, while simultaneously leaving the doctor's hands free to hold the instruments necessary for the examination.

Furthermore, the distribution of the resistance of the retracting instrument on the two joint ends allows the vaginal pressure to be better distributed on the instrument, so as to reduce the need for additional reinforcements.

According to the invention, the instrument can be manufactured as a single piece, significantly reducing the costs of production, which thus does not require additional steps of assembly, or of management of parts in stock, as well as disposal costs, since separation of the different materials is not necessary.

Preferably, according to the invention, each valve presents a cap-shaped end opposite to the side elements of the handle, with the respective concave parts facing each other.

This advantageously allows the insertion of the instrument to be facilitated, making it less uncomfortable for the patient.

Also preferably, the valves can be tapered from the side elements toward the ends, to reduce the physical impact during insertion.

Furthermore, according to the invention, the handle presents a shape adapted to accommodate the palm of a user's hand, so that the first end and the second end project from the side elements in a direction opposite to the valve ends.

This makes the retracting instrument advantageously convenient for a doctor to use, as it allows the fingers to be placed in the correct position so as to exert pressure on the side elements or on the valves without an excessive effort of the hand.

Always according to the invention, the valves can present a curvature in their longitudinal extension, with the concavity facing upwards when inserted, or in any case facing outwards from the volume of the instrument itself.

This advantageously allows the anatomy of the vaginal canal to be followed, without causing discomfort to the patient during the examination.

Finally, according to the invention, the instrument of the invention can be made of TECAPEEK. In this way it is possible to obtain a light and resistant instrument, as well as pleasant to the touch and compatible with the mucosal tissues with which it must come into contact.

### Brief description of the figures

The present invention will now be described, by way of non-limiting example, according to some of its preferred embodiments, and with the aid of the attached figures, in which:
Figure 1 is a front view of the speculum of the invention, in the open position;
Figure 2 is a side view of the speculum of Figure 1, in the open position;
Figure 3 is a rear view of the speculum of Figure 1, in the open position;
Figure 4 is a top view of the speculum of Figure 1, in the open position;
Figure 5 is a perspective view of the speculum of Figure 1, in the open position;
Figure 6 is a top view of the speculum of Figure 1, in the closed position,
Figure 7 is a perspective view of the speculum of Figure 1, in the closed position, held by one hand.

### Detailed description

In the various figures the similar parts will be indicated by the same numerical references.

In the following, reference will be made to the directions of extension (height, width and thickness) of the speculum 100 and of its components in its direction of use, i.e. with the handle 2 vertical and facing outwards and the valves 1 horizontal facing toward the inside of a patient's vagina, so that the valves themselves are joined and retracted moving in a substantially horizontal plane.

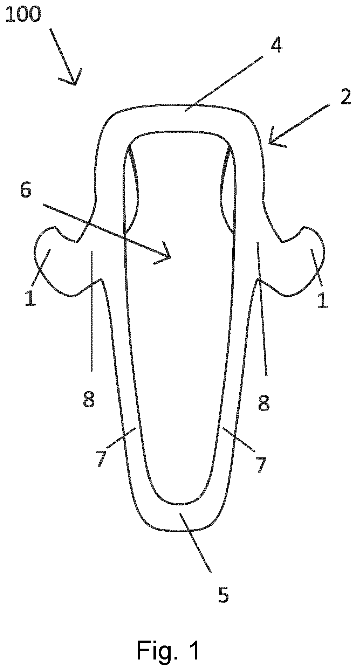

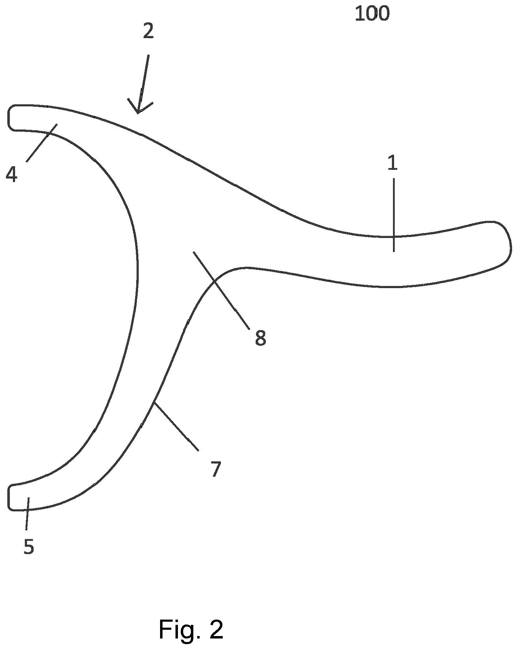

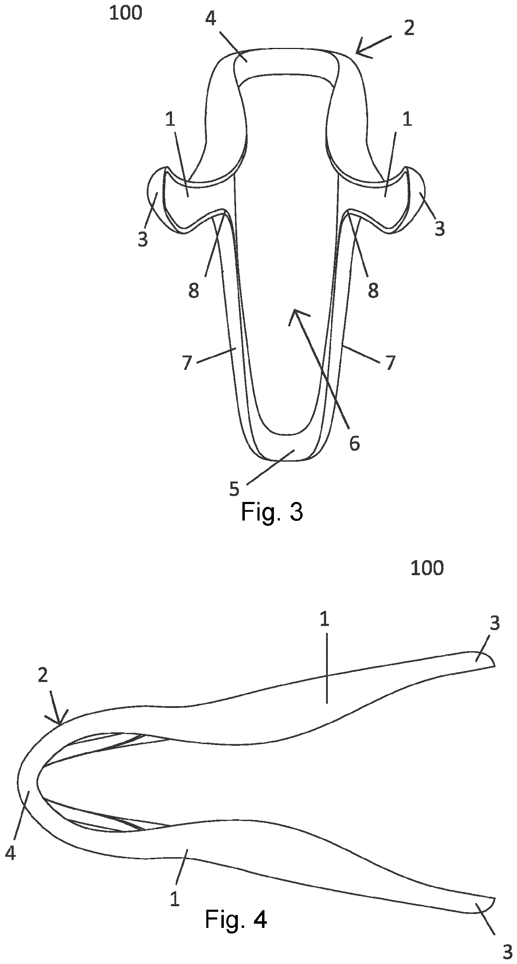

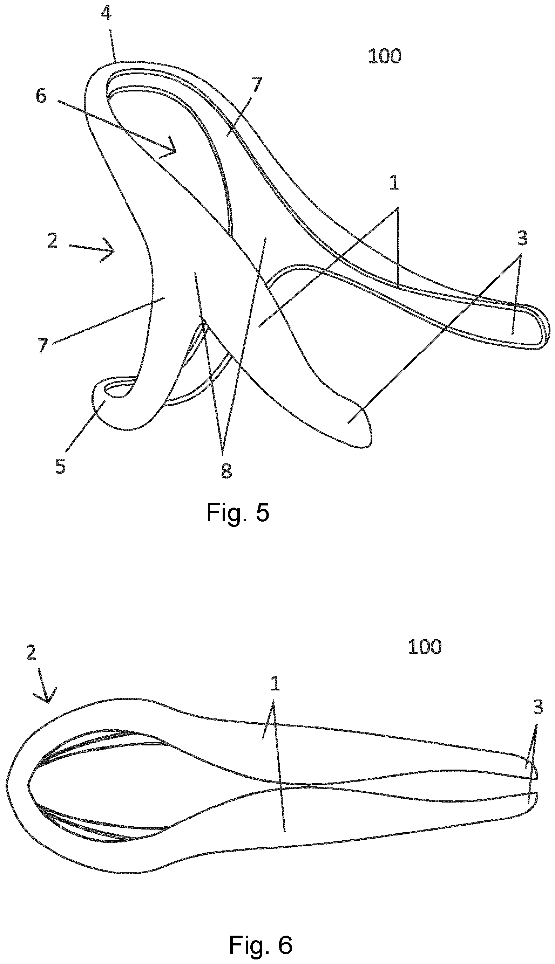

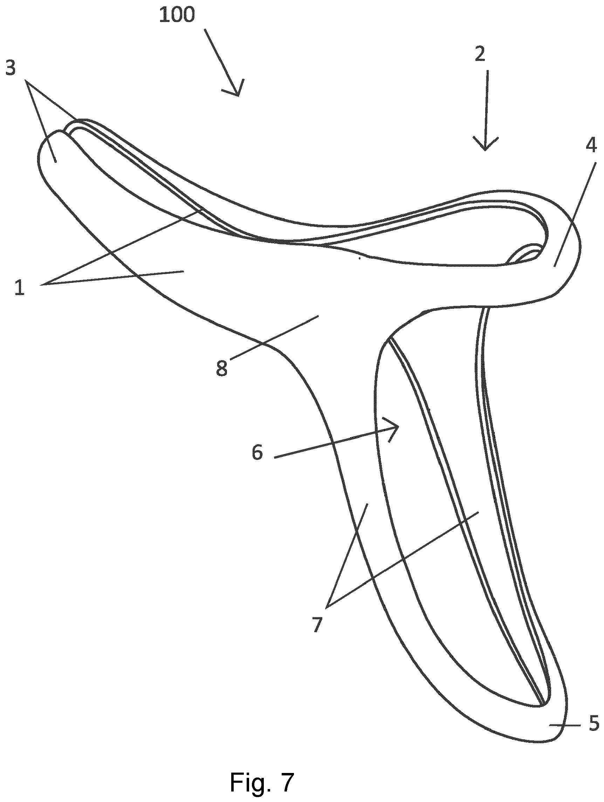

With reference to Figures 1-7, a clamshell speculum 100 is shown, to retract the vagina during a gynecological examination. The device 100 aims to be a valid alternative to the speculum used nowadays, by following the female anatomy it reduces discomfort and maintains the necessary requirements for use by doctors.

In order to do so, the speculum 100 uses a new closing and opening mechanism. The clamshell structure involves the approach of the valves 1, by acting on the handle 2, to reduce the bulk while the speculum 100 itself is entering into the vagina: the ends 3 of the valves 1 are characterized by a curved, cap-like surface so to reduce its thickness at the free end 3 itself, to allow the retractor to advance comfortably.

Furthermore, the height of the valves 1 tends to be reduced at the ends 3, to reduce the physical impact during insertion.

The valves 1 are joined together by means of the handle 2. This handle 2 has a vertical extension such as to make it possible an easy grip on the valves, so as to keep them together by applying pressure with the fingers and the opposite thumb. In particular, the vertical extension corresponds to the size of the palm of a hand. Preferably, the handle has a first end 4, which rests on the palm of the hand, and a second end 5, which can be positioned lower than the hand.

Advantageously, the retracting force is distributed on the two ends 4, 5, making the device 100 stronger.

Between the two ends 4 and 5, the handle is shaped with a recess, so as to accommodate the palm of the hand and to be accommodated in the hollow of the hand itself.

Along this recess there is an opening 6, large enough to allow a hand to pass through and a gynecological examination to be performed when the speculum 100 is inserted into a patient's vaginal canal.

Advantageously, this opening allows the vaginal canal and the cervix to be viewed, which are the areas of interest during the inspection examination.

In this way, the opening 6 is framed by the two ends 4 and 5 of the handle and by side elements 7, preferably parallel to each other and positioned vertically when inserted into the patient's vaginal canal.

It is on the side elements 7 of the handle 2 that the user exerts force to keep the valves 1 closed.

The valves 1 are inserted onto the side elements 7 at a junction point 8, preferably at one third of the height of the handle 2 from its upper end 4.

Starting from the junction point 8, the valves 1 extend substantially perpendicular to the side elements 7, following a curvature with the concavity facing upwards.

The opening of the speculum 100 occurs after being inserted into the cavity: the doctor releases his grip and gradually the valves 1 impose pressure on the vaginal walls.

At this point, the vaginal canal is dilated, and through the opening 6 of the handle 2 it is possible for the gynecologist to observe and conduct tests such as the pap test. Due to the nature of the clamshell mechanism, the gynecologist's hands are advantageously free during the examination, since they do not have to hold the speculum 100 still: this allows them to hold any light source that is necessary to view the inside of the vaginal canal, but also to perform the collection of sample cells in less time.

Another important innovation is the responsiveness and adaptability of the retractor. During the examination women may stiffen or experience a slight muscle spasm: in these cases, discomfort is experienced with a Cusco's Speculum due to the rigidity of the instrument which is locked with an interlocking mechanism to allow it to open.

On the contrary, the speculum 100 of the invention has no locking mechanisms: the valves 1 follow the slight movement performed by the woman's muscles and then return to the open position. All this is possible thanks to the resistance of the material and the bend of the handle 2. Once the inspection examination is concluded, the doctor closes the device 100, by acting on the vertical elements 7 and extracts it.

The instrument has been designed with curved lines and shapes that take into account the needs of women as patients. The speculum 100 of the invention presents itself as a light and harmonious object, far removed from the common perception of the vaginal speculum and its intimidating appearance.

The valves 1 were designed based on studies of female anatomy: these elements present a curvature with the concavity facing upwards to follow the orientation of the vaginal axis. The speculum 100 is made of TECAPEEK, a medical-grade plastic material which will be described below: its excellent mechanical properties make it both resistant and flexible, which are crucial aspects for its use.

One of the main features of the speculum 100 of the invention is that it's composed of a single element made from a material, these qualities allow the production to be facilitated, reducing the costs of materials, processing and disposal, and making the product easily recyclable.

The following materials were considered for the production of a reusable speculum: a high-performance and medical-grade silicone material was sought for the coating. Among the most used is Platinum Silicone: the main chain of this silicone rubber, consisting of an alternation of Si (silicon) and O (oxygen) atoms. Organic R groups are then bonded to the Si atom. This composition provides characteristics of chemical inertness, resistance to high temperatures, but also flexibility, low viscosity and excellent resistance to low temperatures.

Menstrual cups, i.e. applicators to collect menstrual blood as an alternative to sanitary pads, are among the most common applications. Given the application, this material is optimal because it is biocompatible but also durable and reusable: in fact these cups can be washed and reused for approximately 10 years.

A valid alternative to Platinum Silicone is BIOFLEX, a biopolymeric material used in the cylinders of the inflatable penile prosthesis, Titan^{®}, by Coloplast. Key characteristics of this material include its tensile strength and tear resistance.

The substrate, i.e. the rigid part, requires different mechanical properties: polysulfone is a very resistant plastic material that finds application in various medical products, thanks to the FDA registration and the ISO 13485 certification.

PSU, trade name Udel^{®} by Solvay, is the cheapest of the sulfone polymers (Udel ^{®} PSU, s.d.).

The following materials were considered for the production of a disposable speculum.

Polyurethane polymers have been widely used in the medical field for several decades. Depending on the processes and chemical composition, it's possible to design materials for different qualities and, therefore, applications: ranging from the most resistant to the most flexible.

Polyurethanes are employed in the production of female condoms. The use of this material guarantees the product greater resistance and adaptability for individuals allergic to latex.

For the rigid part of the device, acrylic polymers were considered, which occupy an important place in the market of transparent disposable plastics: they are used to produce medical devices that require impact resistance, chemical resistance, biocompatibility and transparency. Some disposable specula are made of polymethyl methacrylate. The sterilization methods used are predominantly cold.

The last step of the research was summarized to identify the suitable material according to the conditions imposed.

TECAPEEK MT is a medical-grade semi-crystalline thermoplastic tested according to ISO 10993 and USP Class VI. Its biocompatibility makes it suitable for contact with both the skin and internal tissues.

This material is characterized by its high mechanical properties: high resistance to stress cracking and to dimensional stability. Its chemical and radiation resistance make it suitable for the hot steam sterilization: TECAPEEK MT does not show any significant loss of mechanical properties, even after more than 1,500 sterilization cycles.

Furthermore, no additional negative influences such as discoloration or color change (yellowing) or even calcification are visible after more than 1,500 cycles.

The main characteristics of the material are as follows.

| | |
|---|---|
| Young's modulus | 4.100 Gpa |
| Poisson's ratio | 0.40 |
| Shear modulus | 2050.000 Mpa |
| Density | 1.310 g/cm³ |
| Damping coefficient | 0.00 |
| Yield stress | 114.000 Mpa |
| Tensile strength | 114.000 Mpa |

During the development of the project, several design constraints were encountered: one of these is the vaginal pressure that the object must withstand during use. In addition to theorizing the product's resistance, it is necessary to verify the behavior under this stress. The pressure exerted by the pelvic floor muscles varies from woman to woman based on different parameters and for this reason there is no definitive data in the literature.

Knowing this data allows the device to be modelled in order to prevent breakages during the examination and to comply with usage requirements. On the other hand, considering excessive pressure can lead not only to the risk of oversizing the object but also to an excessive use of materials and processing. In this sense, an attempt is made to standardize data to allow the speculum to be optimized and mass-produced.

The resistance of the retractor is crucial for its correct use: the tests performed suggest using a material with a high Young's Modulus and better yield strength, and increasing the thickness in some strategic points, such as the bend of the handle. Implementing these modifications is complex since the speculum of the invention is based on a clamshell mechanism with a short lever arm: by increasing the resistance of the object to the vaginal pressure, the effort to be applied to bring the valves closer together (a step necessary to insert the instrument) also increases.

Making uncontrolled changes could lead to the design of a vaginal speculum that resists vaginal pressure but is difficult for the doctor to use. For this reason, a different approach was implemented, which is based on pinch percentile data.

Following the step of calculating the forces, the device of the invention was remodeled taking into account the new requirements: thickness, resistant walls, and a longer arm for applying force.

Subsequently, some simulations were performed using the finite element method with the aim of verifying the resistance value of the material and the model's response to the applied forces.

| Name | Minimum | Maximum |
|---|---|---|
| Security factor | 1.466 | 15 |
| Stress (Von Mises) | 0.07594 Mpa | 78.85 MPa |
| Displacement (Total) | 0.00 mm | 42.48mm |
| Reaction Force (Total) | 0 N | 247.6 N |
| Strain (Equivalent) | 2.92E-05 | 0.02805 |

The results highlight a good resistance of the model at 24 N, when using the Tecapeek. The most critical area is on the handle, near the upper bend; however, the areas where the value of the safety factor is low are localized.

Operationally, to insert the speculum of the invention, the doctor applies pressure along the handle with one or two hands. As a result, the valves are brought closer together and the speculum is inserted.

Once inside the vagina, the doctor releases the pressure exerted on the handle, which allows the instrument to open.

The closing and opening movement is subordinated to a single gesture, which applies pressure on the handle, and which is done by bringing the four fingers and the thumb closer together: this gesture is not sudden but requires a few more seconds and a slight force.

This effort is studied as it encourages or forces the gynecologist to perform the movement more slowly: nevertheless, it requires control to avoid sudden jerks.

The opening and tension exerted depend on the material and thickness along the body of the object. This type of structure allows the gynecologist to keep their hands free to perform the examination: this is essential for performing the pap test, but also for holding the light source necessary for observation.

The invention, as conceived and illustrated herein, is susceptible to numerous modifications and variations, all of which fall within the scope of the inventive concept.

Furthermore, all details may be replaced by other technically equivalent elements.

Finally, the components used, as long as they are compatible with the specific use, as well as the size, may be any according to the requirements and the state of the art.

Where the features and techniques mentioned in any claim are followed by reference signs, such reference signs have been included for the sole aim of increasing the intelligibility of the claims and, accordingly, such reference signs have no limiting effect on the interpretation of each element identified by way of example by these reference signs.

## Claims

1. An instrument (100) for vaginal dilation, comprising two valves (1) joined by a handle (2), presenting a clamshell structure, the handle (2) presenting a central through opening (6) between two side elements (7) joined at a first junction end (4) and at a second junction end (5), the valves (1) being directly connected transversally to the side elements (7), **characterized in that** the valves (1) have a height and thickness both tapered from the side elements (7) toward the free ends (3), in order to reduce the physical impact during insertion.

2. The instrument (100) for vaginal dilation according to claim 1, **characterized in that** it is made in a single piece.

3. The instrument (100) for vaginal dilation according to claim 1 or 2, **characterized in that** each valve (1) presents a free cap-shaped end (3), opposite to the side elements (7) of the handle (2), with the respective concave parts facing each other.

4. The instrument (100) for vaginal dilation according to one of claims 1-3, **characterized in that** the handle (2) presents a vertical extension corresponding to the size of the palm of a hand.

5. The instrument (100) for vaginal dilation according to claim 4, **characterized in that** the handle (2) presents a shape adapted to accommodate the palm of a user's hand, so that the first end (4) and the second end (5) project relative to the side elements (7) in the direction opposite to the ends (3) of the valves (1), so that the first end (4) acts as a support for the palm of the hand, and the second end (5) can be positioned lower than the hand.

6. The instrument (100) for vaginal dilation according to one of claims 1-5, **characterized in that** the valves (1) present in the direction from the side elements (7) toward the free ends (3) a curvature with the concavity facing upward when the instrument (100) is used.

7. The instrument (100) for vaginal dilation according to one of claims 1-6, **characterized in that** the valves (1) are inserted onto the side elements (7) at a junction point (8) at one third of the height of the handle (2) from its first end (4).

8. The instrument (100) for vaginal dilation according to one of claims 1-7, **characterized in that** it is made of Tecapeek.
